(19) Europäisches Patentamt
European Patent Office
Office européen des brevets

(11) Veröffentlichungsnummer : **0 062 903**
**B1**

(12) # EUROPÄISCHE PATENTSCHRIFT

(45) Veröffentlichungstag der Patentschrift : **10.09.86**

(51) Int. Cl.[4] : **C 07 J 1/00**, A 61 K 31/565// C07J21/00

(21) Anmeldenummer : **82103030.1**

(22) Anmeldetag : **08.04.82**

(54) **17-Alpha-Alkyl-17-beta-hydroxy-1-alpha-methyl-4-androsten-3-one, Verfahren zu ihrer Herstellung und pharmazeutische Präparate, die diese Verbindungen enthalten.**

(30) Priorität : **13.04.81 DE 3115996**

(43) Veröffentlichungstag der Anmeldung : **20.10.82 Patentblatt 82/42**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**EP-A- 0 027 961**
**DE-A- 2 046 640**
**DE-B- 1 223 837**
**FR-A- 2 319 367**
**ARZNEIMITTELFORSCHUNG, Band 15, Nr. 10, Oktober 1963, Seiten 1168-1184, Aulendorf, DE. F. NEUMANN et al.: "Über anabole und androgene Wirkungen von in C1 und C1C2 substituierten Steroiden"**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Laurent, Henry, Dr.**
**Glambecker Weg 21**
**D-1000 Berlin 28 (DE)**
Erfinder : **Schleusener, Annerose, Dr.**
**Ilsensteinweg 1B**
**D-1000 Berlin 38 (DE)**
Erfinder : **Albring, Manfred, Dr.**
**Am Dachsbau 34A**
**D-1000 Berlin 27 (DE)**
Erfinder : **Wiechert, Rudolf, Prof., Dr.**
**Petzower Strasse 8a**
**D-1000 Berlin 39 (DE)**

**EP 0 062 903 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 17α-Alkyl-17β-hydroxy-1α-methyl-4-androsten-3-one der allgemeinen Formel I

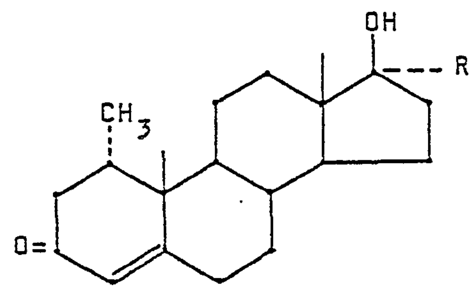

(I)

worin R einen geradkettigen Alkylrest mit 3 bis 8 Kohlenstoffatomen darstellt.

Das 17β-Hydroxy-1α,17α-dimethyl-4-androsten-3-on ist eine seit langem bekannte Verbindung, die sich unter anderem durch eine anabole Wirksamkeit auszeichnet (F. Neumann und R. Wiechert Arzneimittelforschung, *15*, 1965, 1968 ff — speziell 1177 und DE-A-2 046 640). Ähnliche Verbindungen mit anaboler Wirksamkeit sind aus der FR-A-2 319 367 vorbekannt.

Die zum 17β-Hydroxy-1α,17α-dimethyl-4-androsten-3-on homologen 17α-Alkyl-17β-hydroxy-1α-methyl-4-androsten-3-one der allgemeinen Formel I zeichnen sich demgegenüber überraschenderweise bei lokaler Applikation durch eine ausgeprägte antiandrogene Wirksamkeit aus. Bei systemischer Applikation zeigen diese Verbindungen keine ausgeprägte antiandrogene Wirksamkeit, wodurch sie sich vorteilhaft von allen anerkannt wirksamen Handelspräparaten gleicher Wirkungsrichtung unterscheiden.

Darüberhinaus haben die erfindungsgemäßen Verbindungen den vorzug, daß sie die Lipogenese stark hemmen.

Die antiandrogene Wirkung wird wie folgt bestimmt :

Männliche, fertile Hamster im Gewicht von etwa 80 g werden kastriert und mit täglich 0,1 mg Testosteronpropionat subcutan substituiert. Das rechte Ohr wird zweimal täglich mit 0,01 ml einer 3 %igen Lösung der Testsubstanz in Aceton oder Ethanol 3 Wochen lang behandelt. Bei der Kontrollgruppe wird das rechte Ohr nur mit 0,01 ml Lösungsmittel behandelt.

Am 22. Tag werden die Tiere mit Ether getötet, die Samenblase wird präpariert und gewogen. Aus den Ohren werden jeweils definierte Gewebeareale von 3 × 7 mm Kantenlänge ausgestanzt.

Diese werden histologisch weiterbehandelt und die Areale der Talgdrüsen gemessen. Durch Vergleich der Areale der ventralen Seite der Ohren der mit dem Antiandrogen behandelten Tiere mit den gleichen Bereichen der nur mit Lösungsmittel behandelten Tiere erhält man ein Maß für den lokalen antiandrogenen Effekt der Testsubstanz. Die gegenüber einer Kontrollgruppe beobachtete Reduktion des Samenblasengewichts zeigt das Ausmaß der systemischen antiandrogenen Wirksamkeit der Testsubstanz.

Die Beeinflussung der Lipogenese durch die Testsubstanzen wird wie folgt ermittelt :

Männliche, fertile Hamster im Gewicht von etwa 80 bis 100 g werden kastriert und mit täglich 0,1 mg Testosteronpropionat subcutan substituiert. Das rechte Ohr jedes Versuchstieres wird zweimal täglich mit je 0,01 ml einer 1 %igen Lösung der Testsubstanz in Aceton (beziehungsweise bei der Kontrollgruppe nur mit 0,01 ml Lösungsmittel) drei Wochen lang behandelt. Dann werden die Tiere getötet, und aus dem behandelten rechten Ohr sowie dem unbehandelten linken Ohr jeweils ein definiertes Gewebeareal von 8 mm Durchmesser ausgestanzt. Ventrale und dorsale Seite der Ausstanzungen werden entlang des Ohrknorpels voneinander getrennt, unverzüglich in Dulbecco's Modifikation of Eagle's Medium, dem 4 mMol Glutamin und 10 % Kälberserum zugesetzt war und das zur Vermeidung von mikrobieller Kontamination 100 IE/ml Pencillin, 100 μg/ml Streptomycin, 125 μg/ml Kanamycin, 25 IE/ml Nystatin und 10 μg/ml Gentamycin enthält, überführt und eine Stunde lang bei 37 °C inkubiert.

Danach bringt man die Ausstanzungen unter sterilen Bedingungen in frisches Kulturmedium, daß 1 μCi/ml $C^{14}$ markiertes Natriumacetat enthält und inkubiert 4 Studen lang bei 37 °C. Anschließend gibt man die Proben in 2 ml einer Proteolyse-Lösung aus 0,05 Mol Tris-Puffer vom $p_H = 7{,}5$, 0,01 mol Dinatriumethylendiamintetraessigsäure, 0,5 % Natriumdodecylsulfat und 0,1 % Proteinase K (Firma E. Merck A.G. Darmstadt, Bundesrepublik Deutschland), und inkubiert 24 Stunden lang bei 37 °C.

Die so erhaltenen Proben werden einmal mit 5 ml Chloroform und nochmals mit 3 ml Chloroform extrahiert, die vereinigten Chloroformextrakte im Vakuum eingeengt und ihr Gehalt an radiomarkiertem Lipiden im Szintillationszähler ermittelt. Die prozentuale Hemmung der Lipogenese der behandelten Kontrollgruppe wird durch Vergleich mit der nur mit Lösungsmittel behandelten Kontrollgruppe berechnet.

Die nachfolgende Tabelle zeigt die in diesen Tests erhaltenen Ergebnisse.

(Siehe Tabelle Seite 3 f.)

Tabelle

| Nr. | Substanz | Flächenreduktion der Talgdrüsen behandeltes Ohr | Flächenreduktion der Talgdrüsen kontralaterales Ohr | Gewichtsreduktion der Samenblase | Lipogenese Änderung behandeltes Ohr | Lipogenese Änderung kontralaterales Ohr |
|---|---|---|---|---|---|---|
| 1 | 17β-Hydroxy-1α-methyl-17α-propyl-4-androsten-3-on | 60 % | 46 % | 0 % | -45 % | -31 % |
| 2 | 17α-Butyl-17β-hydroxy-1α-methyl-4-androsten-3-on | 64 % | 34 % | 0 % | -40 % | -26 % |

Zur topischen Anwendung können die erfindungsgemäßen Verbindungen mit den üblichen Trägersubstanzen zu Lösungen, Gels, Salben, Pudern oder anderen Präparaten verarbeitet werden. Geeignete Trägersubstanzen sind zum Beispiel Wasser, Ethanol, Propanol, Glycerin, Methylcellulose, Hydroxypropylcellulose, Carboxypolymethylen u.s.w. Das Antiandrogen liegt vorzugsweise in einer Konzentration von 0,05 bis 5,0 Gewichts-%, bezogen auf das gesamtgewicht des Präparates, vor. Die Präparate können zur topischen Behandlung von Erkrankungen wie Akne, Seborrhoe, Alopezie und Hirsutismus verwendet werden.

Die neuen 17α-Alkyl-17β-hydroxy-1α-methyl-4-androsten-3-one können nach einem Verfahren hergestellt werden, welches dadurch gekennzeichnet ist, daß man eine Verbindung der allgemeinen Formel II

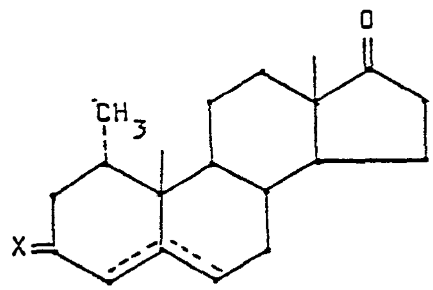

(II)

worin R die obengenannte Bedeutung besitzt, eine der Bindungen .... eine Doppelbindung darstellt und X eine Oxogruppe, eine Oximinogruppe, eine Alkoxygruppe mit 1 bis 4 Kohlenstoffatomen und ein Wasserstoffatom oder eine Alkylendioxygruppe mit 2 bis 6 Kohlenstoffatomen bedeutet, mit einer metallorganischen Verbindung der allgemeinen Formel III

RZ (III),

worin R die obengenannte Bedeutung besitzt und Z ein Alkalimetallion oder ein Magnesiumhalogenidion darstellt, umsetzt.

Dieses Verfahren kann unter Bedingungen durchgeführt werden, die dem Fachmann wohlbekannt sind (H. Laurent und R. Wiechert in : John Fried and John A. Edwards « Organic reactions in Steroid Chemistry » — Volume II — van Nostrand Reinhold Company, New York etc., 1972, 52 ff).

Die nachstehenden Ausführungsbeispiele dienen zur näheren Erläuterung des erfindungsgemäßen Verfahrens.

Beispiel 1

A. Eine Lösung von 7,0 g 17β-Hydroxy-1α-methyl-4-androsten-3-on in 500 ml Benzol versetzt man mit 80 ml 1,2-Ethandiol und 400 mg p-Toluolsulfonsäure und destilliert das sich bildende Wasser innerhalb von 20 Stunden azeotrop ab. Nach dem Abkühlen der Reaktionsmischung wird diese mit 5 ml Pyridin versetzt, mit Wasser gewaschen, über Natriumsulfat getrocknet und im Vakuum eingeengt. Der Rückstand wird an Kieselgel chromatographiert und man erhält mittels Hexan-Ethylacetat-Gradienten (25 bis 30 % Ethylacetat) 6,3 g 3,3-Ethylendioxy-1α-methyl-5-androsten-17β-ol.

B. 3,6 g 3,3-Ethylendioxy-1α-methyl-5-androsten-17β-ol löst man in 100 ml Dichlormethan, versetzt mit 5,8 g Pyridiniumdichromat und rührt 20 Stunden bei Raumtemperatur. Die Reaktionslösung wird mit Wasser gewaschen, getrocknet und im Vakuum eingedampft. Der Rückstand wird an Kieselgel chromatographiert und man erhält mittels Hexan-Ethylacetat-Gradienten (11 bis 16 % Ethylacetat) 2,81 g 3,3-Ethylendioxy-1α-methyl-5-androsten-17-on.

C. Zu einer Lösung von 2,27 g 3,3-Ethylendioxy-1α-methyl-5-androsten-17-on in 80 ml Tetrahydrofuran tropft man unter Rühren bei — 70 °C eine 1,7 molare lösung von Lithiumpropyl in Diethylether. Nach 30 Minuten wird das Reaktionsgemisch in Eiswasser eingegossen. Man extrahiert mit Diethylether, wäscht mit Wasser, trocknet die organische Phase über Natriumsulfat, engt sie im Vakuum zur Trockne ein und erhält 2,40 g 3,3-Ethylendioxy-1α-methyl-17α-propyl-5-androsten-17β-ol.

D. 2,40 g 3,3-Ethylendioxy-1α-methyl-17α-propyl-5-androsten-17β-ol werden in 70 ml Aceton gelöst. Die Lösung wird mit 5 ml Wasser und 460 mg Pyridinium-p-toluolsulfonat versetzt, 3 Stunden lang zum Sieden erhitzt und anschließend im Vakuum eingeengt. Der Rückstand wird in 300 ml Ethylacetat gelöst, die Lösung mit Wasser gewaschen, über Natriumsulfat getrocknet und im Vakuum eingeengt. Das Rohprodukt wird an Kieselgel chromatographiert und man erhält mittels Hexan-Aceton-Gradienten (10 bis 12 % Aceton) nach Umkristallisation aus Diethylether-Benzin 724 g 17β-Hydroxy-1α-methyl-17α-propyl-4-androsten-3-on vom Schmelzpunkt 88 °C.

Beispiel 2

A. Zu einer Lösung von 1,27 g 3,3-Ethylendioxy-1α-methyl-5-androsten-17-on in 50 ml Tetrahydrofuran tropft man bei — 70 °C unter Argon 5 ml einer 1,2 molaren Lösung von Lithiumbutyl in Hexan. Nach 15 Minuten wird das Reaktionsgemisch in Eiswasser eingegossen. Man extrahiert mit Diethylether, wäscht die organische Phase mit Wasser, trocknet sie über Natriumsulfat und engt sie im Vakuum ein. Man erhält so 1,30 g 17α-Butyl-3,3-ethylendioxy-1α-methyl-5-androsten-17β-ol als Rohprodukt.

B. Eine Lösung von 1,30 g 17α-Butyl-3,3-ethylendioxy-1α-methyl-5-androsten-17β-ol in 40 ml Aceton wird mit 3 ml Wasser und 250 mg Pyridinium-p-toluolsulfonat versetzt und 3 Stunden lang zum Sieden erhitzt. Man arbeitet die Reaktionsmischung auf, wie in Beispiel 1 D beschrieben, chromatographiert das erhaltene Rohprodukt an Kieselgel mittels Hexan-Ethylacetat-Gradienten und erhält (bei 16 bis 18 % Ethylacetat) nach Umkristallisation aus Diethylether-Benzin 696 mg 17α-Butyl-17β-hydroxy-1α-methyl-4-androsten-3-on vom Schmelzpunkt 85 °C.

Beispiel 3

A. Eine Lösung von 10,0 g 3,3-Ethylendioxy-1α-methyl-5-androsten-17-on in 100 ml Diethylether wird unter Argon bei — 30 °C innerhalb von 30 Minuten tropfenweise mit 40 ml einer 1,5 molaren Lösung von Lithiumhexyl in Diethylether versetzt. Danach wird die Reaktionsmischung eine Stunde lang bei — 30 °C gerührt und anschließend in Eiswasser gegossen. Man extrahiert mit Diethylether, wäscht die organische Phase mit Wasser, trocknet sie über Natriumsulfat und engt sie im Vakuum ein. Der Rückstand wird an Kieselgel chromatographiert und man erhält mittels Hexan-Ethylacetat-Gradienten (15 bis 25 % Ethylacetat) 5,36 g 3,3-Ethylendioxy-17α-hexyl-1α-methyl-5-androsten-17β-ol.

B. 5,36 g 3,3-Ethylendioxy-17α-hexyl-1α-methyl-5-androsten-17β-ol versetzt man mit 60 ml 90 %iger Essigsäure und erhitzt 15 Minuten lang auf dem Dampfbad. Nach dem Abkühlen wird die Lösung in Eiswasser eingerührt, das ausgefällte Produkt abfiltriert, gewaschen und getrocknet. Das Rohprodukt wird an Kieselgel chromatographiert und man erhält mittels Hexan-Ethylacetat-Gradienten (20 bis 25 % Ethylacetat) nach Umkristallisation aus Diethylether-Diisopropylether 3,47 g 17α-Hexyl-17β-hydroxy-1α-methyl-4-androsten-3-on vom Schmelzpunkt 84 °C.

Beispiel 4

Unter den im Beispiel 3 A beschriebenen Bedingungen werden 8,46 g 3,3-Ethylendioxy-1α-methyl-5-androsten-17-on mit 40 ml einer 1,4 molaren Lösung von Lithiumpentyl in Diethylether umgesetzt, aufgearbeitet und man erhält das 3,3-Ethylendioxy-1α-methyl-17α-pentyl-5-androsten-17β-ol.

Dieses wird unter den Bedingungen des Beispiels 3 B mit 90 %iger Essigsäure hydrolysiert, aufbereitet und man erhält 3,16 g 17β-Hydroxy-1α-methyl-17α-pentyl-4-androsten-3-on vom Schmelzpunkt 144 °C.

**Patentansprüche**

1. 17α-Alkyl-17β-hydroxy-1α-methyl-4-androsten-3-one der allgemeinen Formel I

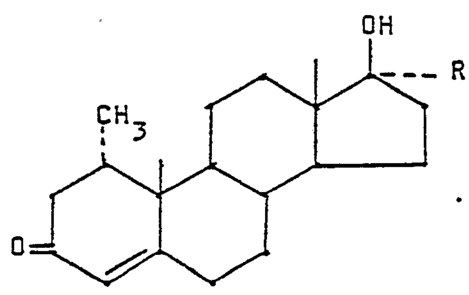

(I)

worin R einen geradkettigen Alkylrest mit 3 bis 8 Kohlenstoffatomen darstellt.

2. 17β-Hydroxy-1α-methyl-17α-propyl-4-androsten-3-on.
3. 17α-Butyl-17β-hydroxy-1α-methyl-4-androsten-3-on.
4. 17β-Hydroxy-1α-methyl-17α-pentyl-4-androsten-3-on.
5. 17α-Hexyl-17β-hydroxy-1α-methyl-4-androsten-3-on.
6. Pharmazeutische Präparate gekennzeichnet durch einen Gehalt an 17α-Alkyl-17β-hydroxy-1α-methyl-4-androsten-3-onen gemäß Anspruch 1 bis 5.
7. Verfahren zur Herstellung von 17α-Alkyl-17β-hydroxy-1α-methyl-4-androsten-3-onen der allgemeinen Formel I

(Siehe Schema Seite 6 f.)

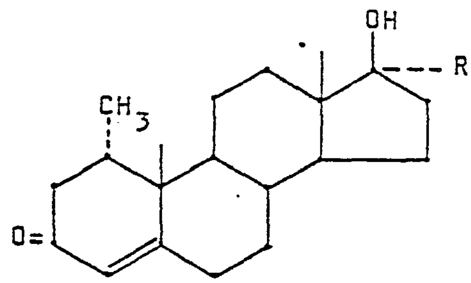

(I)

worin R einen geradkettigen Alkylrest mit 3 bis 8 Kohlenstoffatomen darstellt, dadurch gekennzeichnet, daß man eine Verbindung der allgemeinen Formel II

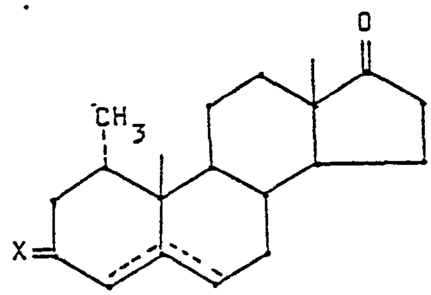

(II)

worin R die obengenannte Bedeutung besitzt, eine der Bindungen .... eine Doppelbindung darstellt und X eine Oxogruppe, eine Oximinogruppe, eine Alkoxygruppe mit 1 bis 4 Kohlenstoffatomen und ein Wasserstoffatom oder eine Alkylendioxygruppe mit 2 bis 6 Kohlenstoffatomen bedeutet,
mit einer metallorganischen Verbindung der allgemeinen Formel III

RZ (III),

worin R die obengenannte Bedeutung besitzt und Z ein Alkalimetallion oder ein Magnesiumhalogenidion darstellt, umsetzt.

**Claims**

1. 17α-Alkyl-17β-hydroxy-1α-methyl-4-androsten-3-ones of the general formula I

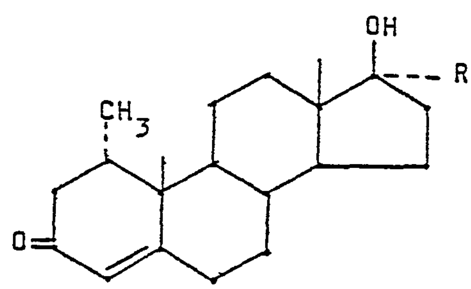

(I)

wherein R is a straight chain alkyl group of 3 to 8 carbon atoms.

2. 17β-Hydroxy-1α-methyl-17α-propyl-4-androsten-3-one.

3. 17α-Butyl-17β-hydroxy-1α-methyl-4-androsten-3-one.

4. 17β-Hydroxy-1α-methyl-17α-pentyl-4-androsten-3-one.

5. 17α-Hexyl-17β-hydroxy-1α-methyl-4-androsten-3-one.

6. Pharmaceutical preparations characterised in that they comprise a 17α-alkyl-17β-hydroxy-1α-methyl-4-androsten-3-one according to claims 1 to 5.

7. Process for the preparation of 17α-alkyl-17β-hydroxy-1α-methyl-4-androsten-3-ones of the general formula I

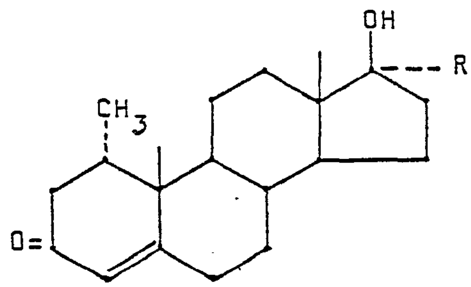

(I)

wherein R is a straight chain alkyl group of 3 to 8 carbons atoms, characterised in that a compound of general formula II

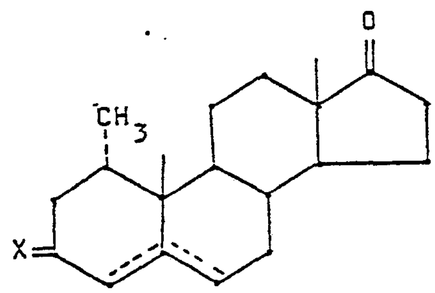

(II)

wherein R has the meaning given above, one of the bonds, ....., is a double bond and X is an oxo group, an oximino group, an alkoxy group of 1 to 4 carbon atoms and a hydrogen atom or an alkylenedioxy group, is reacted with an organometallic compound of general formula III

RZ (III)

wherein R has the meaning given above and Z is an alkali metal ion or a magnesium halide ion.

**Revendications**

1. Alkyl-17α hydroxy-17β méthyl-1α androstène-4 ones-3 répondant à la formule générale I :

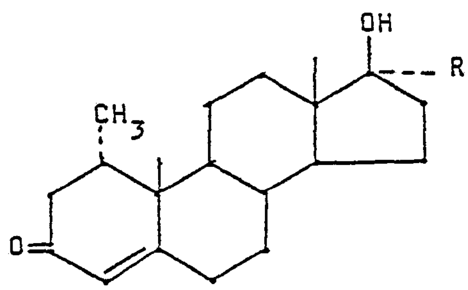

(I)

dans laquelle R représente un radical alkyle linéaire qui contient de 3 à 8 atomes de carbone.

2. Propyl-17α hydroxy-17β méthyl-1α androstène-4 one-3.
3. Butyl-17α hydroxy-17β méthyl-1α androstène-4 one-3.
4. Pentyl-17α hydroxy-17β méthyl-1α androstène-4 one-3.
5. Hexyl-17α hydroxy-17β méthyl-1α androstène-4 one-3.
6. Médicaments caractérisés en ce qu'ils contiennent une alkyl-17α hydroxy-17β méthyl-1α androstène-4 one-3 selon l'une queîconque des revendications 1 à 5.
7. Procédé de préparation d'alkyl-17α hydroxy-17β méthyl-1α androstène-4 ones-3 répondant à la formule générale I :

(Voir dessin page 8)

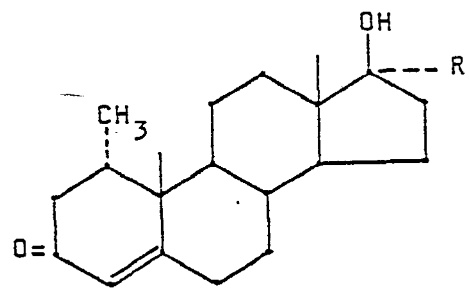

(I)

dans laquelle R représente un radical alkyle linéaire qui contient de 3 à 8 atomes de carbone, procédé caractérisé en ce qu'on fait réagir un composé répondant à la formule générale II :

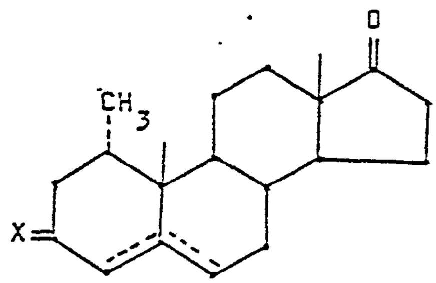

(II)

dans laquelle l'une des liaisons .... représente une double liaison et X représente un radical oxo, un radical hydroxy-imino, un radical alcoxy contenant de 1 à 4 atomes de carbone et un atome d'hydrogène, ou un radical alkylène-dioxy contenant de 2 à 6 atomes de carbone, avec un composé organométallique répondant à la formule générale III :

RZ (III)

dans laquelle R a la signification précédemment donnée et Z représente un ion de métal alcalin ou un ion halogénomagnésien.